# FASCICULE DE BREVET EUROPEEN

(11) **EP 3 169 696 B1**
(45) Date de publication et mention de la délivrance du brevet: **12.06.2019**
(21) Numéro de dépôt: 15753723.4
(22) Date de dépôt: 16.07.2015
(51) Int. Cl.: C07K 1/14, C07K 1/34, C07K 1/36, C07K 14/405

(54) **PROCEDE D'EXTRACTION DES PROTEINES SOLUBLES DE BIOMASSES DE MICROALGUES**
VERFAHREN ZUR EXTRAKTION VON LÖSLICHEN PROTEINEN AUS MIKROALGENBIOMASSE
METHOD FOR EXTRACTING SOLUBLE PROTEINS FROM MICROALGAL BIOMASS

(30) Priorité: 18.07.2014 FR 1456946
(43) Date de publication de la demande: 24.05.2017
(73) Titulaire: Corbion Biotech, Inc., South San Francisco, CA 94080 (US)
(72) Inventeur: PATINIER, Samuel, 59890 Quesnoy sur Deule (FR)
(74) Mandataire: Cabinet Becker et Associés
(86) Numéro de dépôt international: PCT/FR2015/051941
(87) Numéro de publication internationale: WO 2016/009146

(56) Documents cités:
- WO-A1-2011/057406
- CARL SAFI, MICHAËL CHARTON, OLIVIER PIGNOLET, FRANÇOISE SILVESTRE, CARLOS VACA-GARCIA, PIERRE-YVES PONTALIER: "Influence of microalgae cell wall characteristics on protein extractability and determination of nitrogen-to-protein conversion factors", JOURNAL OF APPLIED PHYCOLOGY, vol. 25, 8 août 2012 (2012-08-08), pages 523-529, XP002745231,
- DOUCHA J ET AL: "Influence of processing parameters on disintegration of Chlorella cells in various types of homogenizers", APPLIED MICROBIOLOGY AND BIOTECHNOLOGY, SPRINGER, BERLIN, DE, vol. 81, no. 3, 29 août 2008 (2008-08-29), pages 431-440, XP019654147, ISSN: 1432-0614, DOI: 10.1007/S00253-008-1660-6
- ANJA SCHWENZFEIER ET AL.: BIORESOURCE TECHNOLOGY, vol. 102, 2011, pages 9121-9127, XP28276316, cité dans la demande
- CARL SAFI ET AL: "Morphology, composition, production, processing and applications of Chlorella vulgaris: A review", RENEWABLE AND SUSTAINABLE ENERGY REVIEWS, vol. 35, 1 juillet 2014 (2014-07-01), pages 265-278, XP55138938, ISSN: 1364-0321, DOI: 10.1016/j.rser.2014.04.007

## Description

La présente invention concerne un procédé d'extraction des protéines solubles de la biomasse de microalgues.

Également présentés sont les isolats de protéines de microalgues ainsi obtenus.

### Présentation de l'état de l'art

Il est bien connu de l'homme du métier que les chlorelles sont une source potentielle de nourriture, car elles sont riches en protéines et autres nutriments essentiels.

Elles sont décrites comme renfermant 45% de protéines, 20% de matières grasses, 20% de glucides, 5% de fibres et 10% de minéraux et de vitamines.

Etant donné leur abondance et leur profil en acides aminés, les protéines de microalgues sont ainsi considérées comme une source alternative aux protéines de soja ou de pois en Alimentation.

La fraction protéique peut être également valorisée comme agent fonctionnel dans les industries cosmétiques, voire pharmaceutiques.

Cependant, les développements en applications alimentaires des protéines de microalgues n'ont pas été significatifs, car la présence, dans lesdites fractions, de composés indésirables (telle la chlorophylle) conduit à des changements non souhaités de couleur, goût et structure des compositions alimentaires en contenant.

Pour augmenter leur potentialité en applications alimentaires et pour augmenter également leur valeur commerciale, ces protéines doivent donc être extraites des microalgues sans impacter leur structure moléculaire.

Les techniques « douces » d'extraction seraient donc nécessaires pour isoler les protéines de grandes solubilités et de bonnes propriétés technico-fonctionnelles, mais la rigidité des parois des microalgues, notamment des microalgues vertes, s'y oppose fondamentalement car elle perturbe l'extraction et l'intégrité des protéines intracellulaires.

C'est ainsi qu'au contraire des conditions physiques ou chimiques classiquement « dures » sont mises en oeuvre pour rompre la paroi des microalgues.

De nombreuses études proposent ainsi des technologies de type extraction par solvants organiques ou homogénéisation haute pression.

Dans ces choix technologiques, la dénaturation des protéines n'était cependant pas considérée comme gênante, puisque la plupart de ces méthodes étaient développées pour des finalités d'analyses ou destinées à fournir un substrat pour la digestion enzymatique productrice d'hydrolysats protéiques.

Or, un procédé de désintégration efficace préservant l'intégrité des composants cellulaires se doit de maximaliser non seulement le rendement mais aussi la qualité des produits extraits.

En d'autres termes, un procédé de désintégration optimisé de la paroi doit par exemple éviter :
- la contamination chimique des produits ciblés,
- la mise en oeuvre d'énergie de rupture trop importante ; celle-ci pouvant occasionner une dégradation ou dénaturation irréversible des molécules intracellulaires d'intérêt.

Par ailleurs, pour des productions à grande échelle, il est important que le procédé choisi soit transposable à cette échelle.

Enfin, l'introduction de cette étape de désintégration cellulaire doit être aisée et ne doit pas avoir un impact négatif sur les étapes de procédé/traitement subséquentes.

Toutes ces limitations influencent l'efficacité du procédé de désintégration et par là-même sa consommation énergétique.

C'est la raison pour laquelle la technologie de broyage à billes est préférée, car elle est considérée comme efficace pour libérer les protéines intracellulaires sous leur forme native.

Dans un broyeur à billes, les cellules sont agitées en suspension avec de petites particules sphériques. Le cassage des cellules est provoqué par les forces de cisaillement, le broyage entre les billes, et les collisions avec des billes.

La description d'un broyeur à billes approprié est par exemple faite dans le brevet US 5.330.913. Ces billes cassent les cellules pour en libérer le contenu cellulaire. On obtient alors une suspension de particules de plus petite taille que les cellules d'origine sous la forme d'une émulsion « huile dans eau ».

Cette émulsion est généralement atomisée et l'eau est éliminée, laissant une poudre sèche contenant cependant un mélange hétérogène composé de débris cellulaires, de composés solubles interstitiels et d'huile.

La difficulté à résoudre dans l'emploi de ces technologies de désintégration cellulaire est l'isolement du seul contenu intracellulaire (à l'exclusion des débris membranaires, des sucres, des fibres et des matières grasses) et la préservation, notamment, de la qualité de la charge protéique.

Dans le cas de la microalgue du genre *Tetraselmis sp*, Anja Schwenzfeier et al (Bioresource Technology, 2011, 102, 9121 - 9127) ont proposé un procédé garantissant la solubilité et la qualité de l'aminogramme des protéines isolées et débarrassées des contaminants (telles les substances colorantes) comprenant les étapes suivantes :
- désintégration cellulaire par broyage à billes,
- centrifugation de la suspension de microalgues broyées,
- dialyse du surnageant,
- passage sur résine échangeuse d'ions,
- dialyse de l'éluat,
- décoloration, puis
- lavage et remise en solution.

Cependant, ce procédé de laboratoire (pour traiter 24 g de biomasse) n'est pas transposable à l'échelle industrielle, où le procédé de broyage à billes est plutôt mis en oeuvre pour récupérer une biomasse complète.

Par ailleurs, ce procédé n'est pas adapté aux microalgues qui renferment dans leur biomasse une teneur en lipides non négligeable (par exemple chez *Chlorella protothecoides,* la teneur en lipides est de plus de 15 %).

En effet, même après cette rupture de la paroi cellulaire « relativement douce », le broyat cellulaire se présente sous la forme d'une émulsion complexe « huile dans eau » relativement stable.

L'extraction des composants cellulaires est donc plutôt classiquement réalisée à ce stade par solvant ou par voie mécanique, mais au détriment de leur intégrité.

Une première solution proposée par l'état de l'art, d'ailleurs testée par la société Demanderesse, consiste à coupler le broyage mécanique avec une évaporation afin de tenter de déstabiliser l'émulsion, puis à séparer la fraction grasse par centrifugation.

Cependant, la mauvaise qualité de l'étape de séparation (crémage grossier) rend ce procédé de déphasage peu efficace.

Même l'ajout d'éthanol, recommandé à ce stade (20 - 30 %/brut), s'il améliore la déstabilisation de l'émulsion, ne permet cependant qu'une délipidation de l'ordre de 50 %, et encore à faible rendement.

Par ailleurs, la voie mécanique est particulièrement difficile voire impossible à mettre en oeuvre quand la fraction lipidique est liée à la matrice protéique/polysaccharidique.

Une autre solution propose d'utiliser des solvants neutres. Mais elle présente de grosses contraintes (qualité, sécurité, réglementation...). Le document WO2011/057406 décrit un procédé de préparation d'un isolat protéique de la biomasse de microalgues du genre *Chlorella,* mais ne discute pas la déstructuration de l'émulsion.

### Objet de l'invention

Il résulte qu'il demeure un besoin non satisfait de disposer d'une technologie d'extraction et de stabilisation des composants cellulaires des microalgues d'intérêt, lesdits composants cellulaires étant libérés par un broyage mécanique.

La société Demanderesse a trouvé que ce besoin pouvait être satisfait en proposant un procédé alternatif à ceux connus de l'état de l'art, en combinant un procédé de broyage mécanique des cellules de microalgues avec des étapes de déstructuration de la fraction lipidique produite par un traitement choisi dans le groupe des traitements alcalin et enzymatique, suivi d'une étape de centrifugation.

La fraction soluble délipidée est ensuite clarifiée par microfiltration puis ultrafiltrée pour obtenir l'isolat de protéines.

La présente invention est ainsi relative à un procédé de préparation d'un isolat protéique de la biomasse de microalgues du genre *Chlorella,* comprenant les étapes suivantes :
- fourniture d'une biomasse de microalgues produite par fermentation,
- lavage de la biomasse de manière à éliminer les composés solubles interstitiels, et concentration,
- broyage mécanique de la biomasse lavée et concentrée, conduit dans un système de type broyeur horizontal à billes, pour obtenir une émulsion,
- déstructuration de l'émulsion ainsi obtenue, par l'une des étapes décrites dans les revendications,
- séparation triphasique de manière à séparer la fraction soluble des fractions contenant les lipides et les débris cellulaires,
- récupération, et éventuellement clarification, de la fraction soluble ainsi obtenue, notamment par microfiltration de manière à la débarrasser des insolubles résiduels, afin d'obtenir l'isolat protéique soluble,
- éventuellement, ultrafiltration de la fraction soluble clarifiée sur membrane de seuil de coupure inférieur à 5 kDa, de préférence compris entre 1 et 5 kDa afin d'obtenir un isolat protéique soluble,
- éventuellement, neutralisation à pH 7,
- évaporation, pasteurisation et atomisation dudit isolat protéique.

### Choix de la biomasse de microalgues

De préférence, les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

Dans un mode de réalisation particulier, la souche est *Chlorella protothecoides* (souche UTEX 250 - *The Culture Collection of Algae at the University of Texas at Austin* - USA).

Dans un autre mode de réalisation particulier, la souche est *Chlorella sorokiniana* (souche UTEX 1663 - *The Culture Collection of Algae at the University of Texas at Austin* - USA).

La culture en conditions hétérotrophiques et en l'absence de lumière conduit classiquement à la production d'une biomasse de chlorelles présentant une teneur en protéines (évaluée par la mesure du taux d'azote N x 6,25) de 45 à 70 % en poids de cellules sèches.

Comme il sera exemplifié ci-après, cette culture est réalisée en deux étapes :
- préculture dans un milieu contenant du glucose et de l'extrait de levures pendant 72 h à 28°C sous agitation, puis
- culture pour production de la biomasse proprement dite en glucose et extrait de levures pendant plus de 36 h à 28°C, sous agitation et à pH 6,5 régulé à l'ammoniaque,
ce qui conduit à environ 80 g/L de biomasse à une teneur en protéines (évalué par le N x 6,25) de l'ordre de 52 % en poids de cellules sèches.

La biomasse est ensuite collectée par séparation solide-liquide, par filtration frontale ou tangentielle ou par tout moyen connu par ailleurs de l'homme du métier.

De manière avantageuse, la société Demanderesse recommande ensuite de laver et concentrer la biomasse de manière à éliminer les composés solubles interstitiels par une succession de concentration (par centrifugation) / dilution de la biomasse.

A l'échelle industrielle, il est avantageusement choisi de procéder à une dilution en ligne et séparation par centrifugation en 1 ou 2 étages.

Au sens de l'invention, on entend par « composés solubles interstitiels » tous les contaminants organiques solubles du milieu de fermentation, par exemple les composés hydrosolubles tels les sels, le glucose résiduel, les oligosaccharides de degré de polymérisation (ou DP) 2 ou 3 ou les peptides.

Cette biomasse ainsi purifiée de ses composés solubles interstitiels est ensuite ajustée préférentiellement à une matière sèche comprise entre 15 et 30 % en poids, de préférence à une matière sèche comprise entre 20 et 30 %.

Pour la suite du procédé de l'invention, la biomasse ainsi obtenue peut être utilisée telle quelle, ou perméabilisée thermiquement (par un procédé Haute Température Temps Court - acronyme anglosaxon HTST - par ailleurs développé par la société Demanderesse et protégé dans une de ses demandes non encore publiée) de manière à en libérer le contenu en peptides solubles.

Les protéines résiduelles de cette biomasse peuvent être extraites par la suite des étapes suivantes.

### Broyage de la biomasse

La société Demanderesse recommande d'utiliser la technologie « Bead Mill » (broyeur horizontal à billes).

Plus particulièrement, le broyage peut être réalisé avantageusement selon un procédé que la société Demanderesse a développé et protégé dans une de ses demandes de brevet non encore examinée, dans lequel :
- les billes, en silicate de zirconium, présentent une densité apparente comprise entre 2 et 3,5 kg/l, et
- le taux de remplissage de la chambre de broyage est supérieur ou égal à 80 %.

Quant à la conduite du broyage, elle est réalisée en mode continu, par exemple par passages successifs en série.

La densité des microalgues à broyer est choisie à un taux inférieur à 250 g/l.

A la fin du broyage, on obtient une émulsion.

### Déstructuration de l'émulsion et séparation de ses composantes

La séparation des composantes de l'émulsion afin d'en extraire la fraction peptique ou polypeptidique d'intérêt nécessite une déstructuration/déstabilisation de l'émulsion résultant du broyage cellulaire (mélange complexe de lipides, protéines - peptides et polypeptides - et débris cellulaires).

Cette déstructuration/déstabilisation de l'émulsion est facilitée :
- soit par prédigestion enzymatique, notamment par des protéases spécifiques, par traitement par solvant polaire et/ou par traitement alcalin ménagé ciblant la fraction protéique de l'émulsion,
- soit par ajustement du pH et de la température, par traitement avec un solvant polaire et/ou par digestion enzymatique, notamment de type cellulase, ciblant l'interface avec la fraction lipidique de l'émulsion.

Ainsi, le broyat cellulaire est conditionné dans un réacteur agité équipé d'un module d'agitation peu cisaillant, de manière à limiter l'émulsification tout en permettant un mélange homogène favorisant le traitement spécifique choisi (mise à pH, action de l'enzyme lytique ...).

Par exemple, dans le cas d'un traitement visant à déstabiliser l'émulsion en traitant la fraction protéique en mélange par voie enzymatique, par exemple par une protéase basique, la température et le pH de l'émulsion sont ajustés aux conditions de réaction de ladite protéase :
- la température est ajustée à une valeur supérieure à 30°C, de préférence de l'ordre de 60°C, et
- le pH est ajusté à une valeur supérieure à 7, de préférence de l'ordre de 8 (voire éventuellement de l'ordre de 10 si l'on exploite uniquement l'action du pH).

La durée de la réaction est comprise entre 2 et 8 h.

A la fin de la lyse, un ajout d'éthanol à plus de 5 % (v/v) dans le mélange réactionnel peut éventuellement être effectué comme agent de déstabilisation de l'émulsion (dans le cas d'une forme d'émulsion huile dans eau).

L'émulsion ainsi déstabilisée peut être (partiellement) rompue par séparation triphasique, par exemple par centrifugation.

On obtient ainsi 3 phases :
- un crémage lipidique supérieur,
- une phase aqueuse/composés solubles (et insolubles résiduels) intermédiaires (= solubles « bruts »), et
- un culot concentrant les débris cellulaires.

La fraction soluble est composée essentiellement d'une fraction protéique majoritaire, de sucres solubles, de sels et de globules lipidiques résiduels.

### Séparation membranaire

Pour libérer des peptides et polypeptides, le procédé de l'invention conduit ensuite à l'isolement des protéines d'intérêt, de préférence par fractionnement membranaire.

La société Demanderesse recommande ainsi de procéder en trois étapes :
- récupération et clarification de la fraction soluble ainsi obtenue par microfiltration de manière à la débarrasser des insolubles résiduels,
- ultrafiltration de la fraction soluble clarifiée sur membrane de seuil de coupure inférieur à 5 kDa, de préférence compris entre 1 et 5 kDa, et
- éventuellement neutralisation à un pH compris entre 6 et 8, de préférence à une valeur de 7.

L'exploitation de ces voies permet de purifier les peptides et polypeptides solubles de leurs sels et sucres résiduels.

### Précipitation au pHi

De manière alternative, pour isoler les peptides et polypeptides d'intérêt, il peut être choisi de procéder en trois étapes :
- précipitation des protéines à leur pHi, en ajustant le pH du milieu à une valeur comprise entre 4 et 5,
- centrifugation ou microfiltration afin de récupérer les protéines précipitées, et
- solubilisation dans l'eau à un pH compris entre 6 et 8, de préférence 7.

Il est à noter que ces deux dernières étapes, si elles permettent selon le procédé de l'invention d'obtenir des isolats de protéines ayant une teneur en protéines de plus de 80 %, de préférence de plus de 90 % en poids, conduisent, par leurs modes de mise en oeuvre, à des compositions de nature distincte.

### Obtention de l'isolat sous forme poudre

L'isolat protéique sous forme soluble ainsi obtenu peut être :
- concentré par évaporation,
- pasteurisé et enfin
- atomisé.

L'invention sera mieux comprise à l'aide des exemples qui suivent.

### EXEMPLES

### Exemple 1 : Production de Chlorella protothecoides en fermentation de type fed-batch

La souche utilisée est *Chlorella protothecoides UTEX 250.*

### Pré-culture :

- 500 mL de milieu dans un Erlenmeyer de 2L ;
- Composition du milieu (en g/L) :

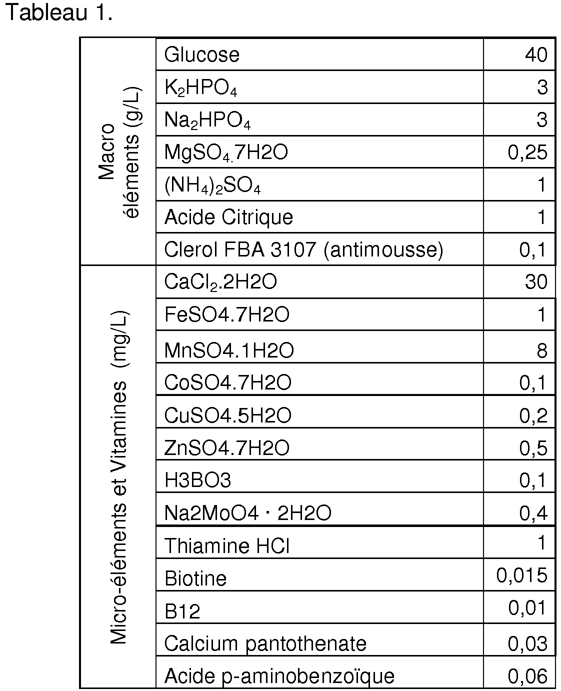

L'incubation se déroule dans les conditions suivantes : durée : 72 h ; température : 28°C ; agitation : 110 rpm (Incubateur Infors Multitron).

La pré-culture est ensuite transférée dans un fermenteur de 30L de type Sartorius.

### Culture pour production de biomasse :

Le milieu est le suivant :

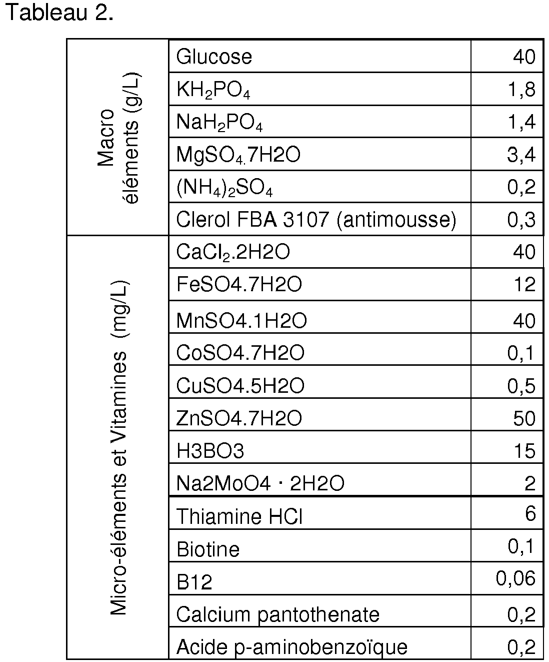

Le volume initial (Vi) du fermenteur est ajusté à 17 L après ensemencement. Il est porté à 20 à 25 L environ en final.

Les paramètres de conduite de la fermentation sont les suivants :

**Tableau 3.**

| | |
|---|---|
| Température | 28 °C |
| pH | 5,0 - 5,2 par NH₃ 28% w/w |
| pO₂ | 20% +/- 5% (maintenue par agitation) |
| Agitation | 300 RPM mini |
| Débit d'air | 15 L/min |

Lorsque la concentration résiduelle en glucose tombe en dessous de 10 g/L, un apport de glucose sous forme d'une solution concentrée à 800 g/L environ est réalisé de façon à maintenir la teneur en glucose entre 0 et 20 g/L dans le fermenteur.

### Résultats

En 40h, on obtient 80 g/L de biomasse contenant 52 % de protéines.

### Exemple 2. Broyage de la biomasse de Chlorella protothecoides et récupération de la fraction soluble - déstructuration de l'émulsion par traitement de la fraction peptidique et polypeptidique

La biomasse obtenue selon l'exemple 1 est lavée et concentrée par centrifugation de manière à être amenée à une matière sèche de 220 g/l et à une pureté de plus 90 % (pureté définie par le rapport entre la matière sèche de la biomasse sur matière sèche totale).

Elle est ensuite broyée par broyage à billes (broyeur horizontal) avec des billes de silicate de zirconium (0,6mm de diamètre, densité apparente de 2,4).

La biomasse broyée est alors agitée dans un réacteur équipé avec une hélice marine et chicanes. La température est ajustée à 60°C et le pH à 8 avec la potasse. Une protéase basique en combinaison avec une cellulase sont ajoutées avec le maintien de ces conditions réactionnelles sur une durée de 6h.

L'émulsion est alors centrifugée sur centrifugeuse triphasique qui permet d'obtenir 3 phases : un crémage lipidique supérieur, une phase aqueuse/composés solubles (et insolubles résiduels) intermédiaires (= solubles « bruts ») et un culot concentrant les débris cellulaires.

La fraction de solubles bruts est clarifiée par microfiltration. Le perméat « P1 » de microfiltration titre entre 55 et 70 % de peptides et protéines (exprimés en acides aminés totaux), et est ensuite ultrafiltré sur membrane de seuil de coupure < 5 kDa.

Le rétentat « R2 » d'ultrafiltration ainsi obtenu contient plus de 80 % de peptides présentant un poids moléculaire supérieur ou égal à 5 kDa.

Le perméat « P2 » contient quant à lui des peptides présentant un poids moléculaire inférieur à 5 kDa et des oligosaccharides et sels résiduels.

Ce perméat « P2 » peut alors être notamment filtré sur membrane d'osmose inverse (présentant un taux de réjection en NaCI de 93 %), de manière à obtenir :
o un rétentat « R3 » contenant des peptides présentant un poids moléculaire inférieur à 5 kDa et des oligosaccharides de DP 2, tel le saccharose ; et
o un perméat « R3 » contenant des oligosaccharides de DP1, des sels, des acides aminés libres et des acides organiques.

L'isolat protéique « R2 » est alors :
- neutralisé à pH 7 à la potasse,
- concentré par évaporation à 35% de matière sèche (MS),
- pasteurisé puis
- atomisé.

### Exemple 3. Broyage de la biomasse de Chlorella orotothecoides et récupération de la fraction soluble - déstructuration de l'émulsion par traitement de la fraction lipidique

Selon le même enchainement que l'exemple 2, la biomasse broyée est agitée dans un réacteur équipé avec une hélice marine et chicanes. La température est ajustée à 50°C sans ajustement de pH (naturellement entre 5 et 6).

Une cellulase présentant une activité optimale dans cette plage de pH et de température est ajoutée avec le maintien de ces conditions réactionnelles sur une durée de 6h.

En fin de réaction, le pH est ajusté à 8 préalablement à la séparation en 3 phases.

La suite des opérations est décrite dans l'exemple 2.

## Revendications

1. Procédé de préparation d'un isolat protéique de la biomasse de microalgues du genre *Chlorella,* **caractérisé en ce qu'**il comprend les étapes suivantes :
- fourniture d'une biomasse de microalgues produite par fermentation,
- lavage de la biomasse de manière à éliminer les composés solubles interstitiels et concentration,
- broyage mécanique de la biomasse lavée et concentrée, conduit dans un système de type broyeur horizontal à billes, pour obtenir une émulsion,
- déstructuration de l'émulsion ainsi obtenue,
- séparation triphasique de manière à séparer la fraction soluble des fractions contenant les lipides et les débris cellulaires,
- récupération de la fraction soluble ainsi obtenue afin d'obtenir l'isolat protéique soluble, puis
- évaporation, pasteurisation et atomisation dudit isolat protéique,
la déstructuration de l'émulsion étant réalisée :
- soit par prédigestion enzymatique, par traitement par solvant polaire et/ou par traitement alcalin ménagé ciblant la fraction protéique de l'émulsion,
- soit par ajustement du pH et de la température, par traitement avec un solvant polaire et/ou par digestion enzymatique ciblant l'interface avec la fraction lipidique de l'émulsion.

2. Procédé selon la revendication 1, **caractérisé en ce que** les microalgues du genre *Chlorella* sont choisies dans le groupe constitué de *Chlorella vulgaris*, *Chlorella sorokiniana* et *Chlorella protothecoides,* et sont plus particulièrement *Chlorella protothecoides.*

3. Procédé selon l'une quelconque des revendications 1 et 2, **caractérisé en ce que** la séparation triphasique de l'émulsion déstructurée est réalisée par centrifugation.

4. Procédé selon l'une quelconque des revendications 1 à 3, **caractérisé en ce que** l'obtention de l'isolat protéique soluble à partir de la fraction soluble est réalisée par :
- clarification de ladite fraction soluble par microfiltration de manière à la débarrasser des insolubles résiduels,
- ultrafiltration de la fraction soluble clarifiée sur membrane de seuil de coupure inférieur à 5 kDa, et
- Eventuellement, neutralisation à un pH compris entre 6 et 8, de préférence à une valeur de 7.

5. Procédé selon l'une quelconque des revendications 1 à 3, **caractérisé en ce que** l'obtention de l'isolat protéique soluble à partir de la fraction soluble est réalisée par :
- précipitation des protéines à leur pHi, en ajustant le pH du milieu à une valeur comprise entre 4 et 5,
- centrifugation ou microfiltration afin de récupérer les protéines précipitées, et
- solubilisation dans l'eau à un pH compris entre 6 et 8, de préférence 7.

## Patentansprüche

1. Verfahren zur Herstellung eines Proteinisolats aus der Biomasse von Mikroalgen der Gattung *Chlorella,* **dadurch gekennzeichnet, dass** es die folgenden Schritte umfasst:
- Bereitstellen einer durch Fermentation erzeugten mikroalgischen Biomasse,
- Waschen der Biomasse, um die interstitiell löslichen Verbindungen und Konzentration zu eliminieren,
- mechanisches Mahlen der gewaschenen und konzentrierten Biomasse, durchgeführt in einem horizontalen Perlmühlen-System, um eine Emulsion zu erhalten,
- Destrukturieren der so erhaltenen Emulsion,
- Dreiphasentrennung, um die lösliche Fraktion von den Fraktionen zu trennen, die Lipide und Zellabfälle enthalten,
- Gewinnen der so erhaltenen löslichen Fraktion, um das lösliche Proteinisolat zu erhalten, und dann
- Verdampfen, Pasteurisieren und Zerstäuben des Proteinisolats,
wobei die Destrukturierung der Emulsion durchgeführt wird:
• entweder durch enzymatische Vorverdauung, durch Behandlung mit polarem Lösungsmittel und/oder durch kontrollierte alkalische Behandlung, die auf die Proteinfraktion der Emulsion abzielt,
• oder durch Einstellen des pH-Wertes und der Temperatur, durch Behandeln mit einem polaren Lösungsmittel und/oder durch enzymatischen Abbau, der auf die Grenzfläche mit der Lipidfraktion der Emulsion abzielt.

2. Das Verfahren nach Anspruch 1, **dadurch gekennzeichnet, dass** die Mikroalgen der Gattung *Chlorella* ausgewählt sind aus der Gruppe bestehend aus *Chlorella vulgaris*, *Chlorella sorokiniana* und *Chlorella protothecoides,* vor allem aber aus *Chlorella protothecoides*

3. Das Verfahren nach einem der Ansprüche 1 und 2, **dadurch gekennzeichnet, dass** die Dreiphasentrennung der destrukturierten Emulsion durch Zentrifugation durchgeführt wird.

4. Das Verfahren nach einem der Ansprüche 1 bis 3, **dadurch gekennzeichnet, dass** das lösliche Proteinisolat aus der löslichen Fraktion erhalten wird durch:
- Klären der löslichen Fraktion durch Mikrofiltration, um restliche unlösliche Substanzen daraus zu entfernen,
- Ultrafiltration der geklärten löslichen Fraktion auf einer Membran mit einer Cut-off-Schwelle von weniger als 5 kDa,
- gegebenenfalls Neutralisieren bei einem pH-Wert zwischen 6 und 8, vorzugsweise bei einem Wert von 7.

5. Das Verfahren nach einem der Ansprüche 1 bis 3, **dadurch gekennzeichnet, dass** das lösliche Proteinisolat aus der löslichen Fraktion erhalten wird durch:
- Ausfällen der Proteine bei ihrem pl, durch Einstellen des pH-Wertes des Mediums auf einen Wert zwischen 4 und 5,
- Zentrifugation oder Mikrofiltration, um die ausgefällten Proteine zu gewinnen, und
- Lösen in Wasser bei einem pH-Wert zwischen 6 und 8, vorzugsweise 7.

## Claims

1. A method for preparing a protein isolate from the biomass of microalgae of the *Chlorella* genus, **characterized in that** it comprises the following steps:
- providing a microalgal biomass produced by fermentation,
- washing the biomass so as to eliminate the interstitial soluble compounds and concentration,
- mechanical milling of the washed and concentrated biomass, carried out in a horizontal bead mill type system, to obtain an emulsion,
- destructuring the emulsion obtained in this way,
- triphase separation so as to separate the soluble fraction from the fractions containing lipids and cell debris,
- recovering the soluble fraction obtained in this way in order to obtain the soluble protein isolate, then
- evaporation, pasteurization and atomization of said protein isolate,
wherein destructuring the emulsion is performed:
• either by enzymatic predigestion, by treatment with polar solvent and/or by controlled alkaline treatment targeting the protein fraction of the emulsion,
• or by adjusting the pH and the temperature, by treatment with a polar solvent and/or by enzymatic digestion targeting the interface with the lipid fraction of the emulsion.

2. The method as claimed in claim 1, **characterized in that** the microalgae of the *Chlorella* genus are chosen from the group consisting of *Chlorella vulgaris, Chlorella sorokiniana* and *Chlorella protothecoides,* and are more particularly *Chlorella protothecoides.*

3. The method as claimed in any one of claims 1 and 2, **characterized in that** the triphase separation of the destructured emulsion is carried out by centrifugation.

4. The method as claimed in any one of claims 1 to 3, **characterized in that** the soluble protein isolate is obtained from the soluble fraction by:
- clarifying said soluble fraction by microfiltration so as to remove residual insoluble substances therefrom,
- ultrafiltration of the clarified soluble fraction on a membrane with a cut-off threshold of less than 5 kDa,
- optionally, neutralizing at a pH of between 6 and 8, preferably at a value of 7.

5. The method as claimed in any one of claims 1 to 3, **characterized in that** the soluble protein isolate is obtained from the soluble fraction by:
- precipitating the proteins at their pi, by adjusting the pH of the medium to a value of between 4 and 5,
- centrifugation or microfiltration in order to recover the precipitated proteins, and
- dissolving in water at a pH of between 6 and 8, preferably 7.
